# EUROPÄISCHE PATENTSCHRIFT

(11) **EP 0 880 494 B1**
(45) Veröffentlichungstag und Bekanntmachung des Hinweises auf die Patenterteilung: **03.05.2000**
(21) Anmeldenummer: 97904422.9
(22) Anmeldetag: 12.02.1997
(51) Int. Cl.: C07C 45/50, C07C 45/80, C07C 29/16, B01J 31/40

(54) **VERFAHREN ZUR HERSTELLUNG VON ALDEHYDEN DURCH HYDROFORMYLIERUNG MIT EINEM RHODIUMKATALYSATOR UND WIEDERGEWINNUNG DES RHODIUMKATALYSATORS DURCH EXTRAKTION**
METHOD OF PREPARING ALDEHYDES BY HYDROFORMYLATION WITH A RHODIUM CATALYST AND RECOVERY OF THE RHODIUM CATALYST BY EXTRACTION
PROCEDE DE FABRICATION D'ALDEHYDES PAR HYDROFORMYLATION AU MOYEN D'UN CATALYSEUR AU RHODIUM ET DE RECUPERATION DE CE CATALYSEUR PAR EXTRACTION

(30) Priorität: 14.02.1996 DE 19605435
(43) Veröffentlichungstag der Anmeldung: 02.12.1998
(73) Patentinhaber: BASF AKTIENGESELLSCHAFT, 67056 Ludwigshafen (DE)
(72) Erfinder: GEISSLER, Bernhard, D-67281 Kirchheim (DE); KNEUPER, Heinz-Josef, D-68163 Mannheim (DE); RÖPER, Michael, D-67157 Wachenheim (DE); PACIELLO, Rocco, D-67098 Bad Dürkheim (DE); OPPENLÄNDER, Knut, D-67061 Ludwigshafen (DE); GÜNTHER, Wolfgang, D-67582 Mettenheim (DE)
(86) Internationale Anmeldenummer: EP9700635
(87) Internationale Veröffentlichungsnummer: WO9730016

(56) Entgegenhaltungen:
- EP-A- 0 695 734
- DE-A- 4 408 950
- DATABASE WPI Section Ch, Week 9047 Derwent Publications Ltd., London, GB; Class A97, AN 90-353272 XP002029907 & SU 1 535 603 A (KAZA CHEM-TECHN INS) , 15.Januar 1990

## Beschreibung

Die vorliegende Erfindung betrifft ein Verfahren zur Herstellung von Alkoholen und/oder Aldehyden durch die Hydroformylierung von Olefinen mit mehr als 3 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion durch Extraktion mit einer wäßrigen Lösung eines wasserlöslichen komplexbildenden Polymers, Isolierung des Hydroformylierungsprodukts aus der organischen Phase, sowie Vorcarbonylierung des wäßrigen, das Rhodium enthaltenden Extrakts unter Zusatz einer mit Wasser im wesentlichen nicht mischbaren organischen Flüssigkeit und Rückführung der organischen Phase in die Hydroformylierung.

Die Hydroformylierung von Olefinen mit Kohlenmonoxid und Wasserstoff in Gegenwart von Übergangsmetall-Katalysatoren ist wohl bekannt. Während α-Olefine sehr gut mit rhodiumhaltigen, phosphinmodifizierten Katalysatoren hydroformylierbar sind (vgl. J. Falbe, Ed: New Syntheses With Carbon Monoxide, Springer, Berlin 1980, S. 55ff), ist dieses Katalysatorsystem für interne und interne, verzweigte Olefine sowie für Olefine mit mehr als 7 Kohlenstoffatomen wenig geeignet (vgl. Falbe, S. 95ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogenen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

Hingegen lassen sich interne und interne, verzweigte Olefine vorteilhaft mit sogenanntem "nacktem" Rhodium hydroformylieren, d.h. mit homogenen im Hydroformylierungsmedium gelösten Rhodiumverbindungen, die nicht mit phosphorhaltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe, S. 38ff). Die Begriffe "nacktes Rhodium" oder "nackte Rhodium-Katalysatoren" werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphin- oder Phosphit-Liganden, modifiziert sind. Als Liganden in diesem Sinne werden nicht Carbonyl- oder Hydrido-Liganden verstanden. Es wird in der Fachliteratur (s. Falbe, S. 38ff) angenommen, daß die Rhodium-Verbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungsreaktionszone nebeneinander ablaufenden Chemismen nicht eindeutig bewiesen ist. Lediglich der Einfachheit halber machen wir auch hier von dieser Annahme Gebrauch, ohne daß dadurch eine Beschränkung bewirkt werden soll, falls sich zukünftig einmal eine andere Rhodiumspezies als die genannte, als die eigentlich katalytisch aktive, herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonyl-Verbindungen, wie Rh₄(CO)₁₂ und Rh₆(CO)₁₆ oder aus Organo-Rhodium-Verbindungen, wie Rhodiumdicarbonylacetylacetonat, Cyclooctadien-Rhodium-acetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen: US-A4400547; DE-A3338340; DE-A2604545; WO 82/03856; Chem.Ber. 102, 2238 (1969); Tetrahedron Lett. 29, 3261 (1968); Hydrocarbon Process. 85-86 (1975).

Allerdings hat auch die Hydroformylierung mit "nacktem" Rhodium den Nachteil, daß sich der thermolabile Rhodium-Katalysator (vgl. US-A4 400547) infolge der thermischen Belastung bei der destillativen Aufarbeitung des Hydroformylierungsproduktes teilweise zu metallischem Rhodium zersetzt, das sich an den Wandungen des Reaktors und der Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

Zur Lösung dieser Probleme sind in der Literatur zahlreiche Verfahren vorgeschlagen worden, von denen das Verfahren der DE-A 42 30 871 (dort auch weitere Vorliteratur zitiert) eine brauchbare Lösung in Form einer Extraktion des Katalysators mit sulfonierten, stickstoffhaltigen niedermolekularen Komplexbildnern und Rückführung des extrahierten Katalysators darstellt. Dieses Verfahren ist weiter verbessert im Verfahren der WO 95/25080. Dort ist zusätzlich eine Vorcarbonylierung des Extrakts vor Rückführung in die Hydroformylierung beschrieben.

Ein dem Verfahren WO 95/250080 ähnliches Verfahren wird in EP-A 695 734 beschrieben, wobei der Katalysator mittels einer wässrigen Lösung phosphorhaltiger Komplexbildner, ausgewählt aus der Gruppe der einfach oder mehrfach sulfonierten und/oder carboxylierten Mono- oder Oligophosphane, aus dem Hydroformylierungsaustrag extrahiert wird. Die in EP-A 695 734 offenbarten phosphorhaltigen Komplexbildner sind allesamt niedermolekulare Verbindungen.

Während durch diese Verbesserung das Verfahren in der technischen Ausführung bereits eine technische Anwendung gestattet, blieb der Erfolg der Extraktion selbst unbefriedigend: zum einen war der Extraktionsgrad sowie die Reversibilität der Komplexbildung und Komplexaufspaltung in der Vorcarbonylierung unbefriedigend und zum anderen sind die in den beiden vorgenannten Schriften beschriebenen sulfonsäuregruppenhaltigen niedermolekularen Komplexbildner schwer zugänglich und teuer, so daß die Aufgabe bestand Extraktionsmittel vorzuschlagen, die
- leicht zugänglich sind
- wasserlöslich sind, ohne in wesentlichen Mengen in die organische Phase überzugehen und
- starke komplexbindende Eigenschaften haben, so daß eine gute Extraktionswirkung gewährleistet ist.

Diese Aufgabe wurde erfindungsgemäß gelöst mit einem Verfahren zur Herstellung von Aldehyden oder Aldehyden und Alkoholen durch Hydroformylierung von Olefinen mit mehr als 3 Kohlenstoffatomen, umfassend eine Hydroformylierungsstufe, in der man mittels eines in einem homogenen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodiumkatalysators mit einer wäßrigen Lösung eines Komplexbildners, Isolierung des Alkohols und/oder Aldehyds aus dem extrahierten Hydroformylierungsaustrag, Vorcarbonylierung des wäßrigen rhodiumhaltigen Extrakts in Gegenwart von Kohlenmonoxid, Synthesegas oder eines Kohlenmonoxid enthaltenden Gasgemisches bei einem Druck von 50 bis 1000 bar und einer Temperatur von 50 bis 180°C, Trennung des Austrags der Vorcarbonylierung in eine dem Hauptteil des Rhodiums enthaltende organische Phase und eine den Komplexbildner enthaltende wäßrige Phase und Rückführung der organischen Phase in die Hydroformylierungsstufe, wobei man den Rhodiumkatalysator aus dem Austrag der Hydroformylierungsstufe mit einer wäßrigen Lösung eines sulfonsäuregruppenfreien, wasserlöslichen Polymeren, das mit dem Rhodiumkatalysator einen wasserlöslichen Komplex bildet, extrahiert und aus dem gebildeten wasserlöslichen Komplex das Rhodium in der Vorcarbonylierung als lipophile Rhodiumcarbonylverbindung herauslöst. Die wasserlöslichen Polymere sind insbesondere ausgewählt aus der Gruppe bestehend aus
(a) Polyacrylsäuren, die teilweise oder vollständig neutralisiert sein können, mit einer mittleren Molmasse von mehr als 500 g/ mol
(b) Maleinsäure-Copolymeren mit einer mittleren Molmasse von mehr als 3000 g/mol,
(c) einfach oder mehrfach phosphonomethylierte Polyvinylaminen mit einer mittleren Molmasse von mehr als 800 g/mol,
(d) einfach oder mehrfach phosphonomethylierte Polyethyleniminen mit einer mittleren Molmasse von mehr als 200 g/mol, und/oder
(e) einfach oder mehrfach phosphonomethylierte Polyacrylamiden mit einer mittleren Molmasse von mehr als 800 g/mol.

Als Komplexbildner, die mit dem im Austrag der Hydroformylierungsreaktion gelösten Rhodium-Katalysator wasserlösliche Komplexe bilden, kommen vorzugsweise stickstoffhaltige phosphonomethylierte Komplexbildner in Betracht.

Als erfindungsgemäß zu verwendende, wasserlösliche, phosphonomethylierte Polymere kommen insbesondere phosphonomethylierte Polyimine im wesentlichen enthaltend oder bestehend aus Einheiten der allgemeinen Formel 1 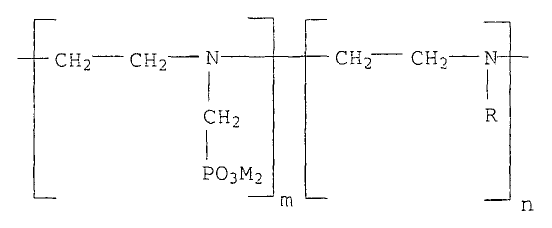 in Betracht, in der jedes M unabhängig für Wasserstoff, Ammonium, für ein Kation eines einwertigen Metallions oder für das Äquivalent eines mehrwertigen Metalls, insbesondere eines Metalls ausgewählt aus der Gruppe bestehend aus Natrium, Kalium, Calzium und Magnesium, steht, wobei das mittlere Molekulargewicht des wasserlöslichen Polymers von 200 bis 2000000 g/mol beträgt, das Verhältnis von m zu (n+m) von 0,01 bis 1, bevorzugt von 0,5 bis 1, jeder Rest R unabhängig Wasserstoff, Alkyl, Aryl, Hydroxyalkyl, Carboxyalkyl sein kann, ferner insbesondere wasserlösliche, phosphonomethylierte Polyvinylamine im wesentlichen enthaltend oder bestehend aus Einheiten der allgemeinen Formel 2 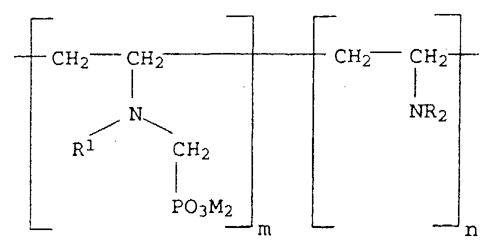 in der jedes M unabhängig für Wasserstoff, Ammonium, ein Kation eines einwertigen Metalls oder für das Äquivalent eines mehrwertigen Metalls, insbesondere eines Metalls ausgewählt aus der Gruppe bestehend aus Natrium, Kalium, Calzium und Magnesium, steht, wobei das mittlere Molekulargewicht des wasserlöslichen Polymers von 800 bis 5000000 g/mol und das Verhältnis von m zu (n+m) von 0,01 bis 1, bevorzugt von 0,5 bis 1 beträgt, R¹ für R oder CH₂PO₃M₂ steht und jedes R unabhängig Wasserstoff, Alkyl, Aryl, Hydroxyalkyl oder Carboxyalkyl sein kann, ferner insbesondere wasserlösliche, phosphonomethylierte Polyacrylamide im wesentlichen enthaltend oder bestehend aus Einheiten der allgemeinen Formel 3 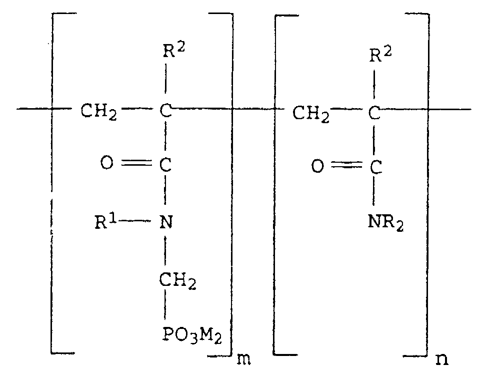 in der jedes M unabhängig für Wasserstoff, für Ammonium, für ein Kation eines einwertigen Metalls oder für das Äquivalent eines mehrwertigen Metalls, insbesondere eines Metalls ausgewählt aus der Gruppe bestehend aus Natrium, Kalium, Calzium und Magnesium steht, wobei das mittlere Molekulargewicht des wasserlöslichen Polymers von 800 bis 5000000 g/mol und das Verhältnis von m zu (n+m) von 0,01 bis 1, bevorzugt von 0,5 bis 1 beträgt, R¹ für R oder CH₂PO₃M₂, R² für Wasserstoff oder Alkyl steht und wobei jedes R unabhängig Wasserstoff, Alkyl, Aryl, Hydroxyalkyl oder Carboxyalkyl sein kann.

Aus *J. Org. Chem.*, Band 31, 1603-1607 (1966) ist die Phosphonomethylierung von Aminen bekannt. Sie verläuft in Analogie zu der Mannich-Reaktion, indem man Amine, die mindestens eine N-H-Gruppe aufweisen im sauren pH-Bereich mit phosphoriger Säure und Formaldehyd umsetzt.

Aus *Makromol. Chem.*, Band 128, 229 (1969) ist bekannt, daß auch Polymere (Polyethylenimin) mit Formaldehyd und phosphoriger Säure phosphonomethyliert werden können, bei denen methylphosphorige Säuregruppen am Polymergerüst fixiert sind.

Es hat sich gezeigt, daß es für das erfindungsgemäße Verfahren von Vorteil ist, wenn der Phosphonomethylierungsgrad des stickstoffhaltigen Polymeren (Verhältnis von m zu (m+n)) möglichst hoch ist, da mit steigendem Phosphonomethylierungsgrad die Wasserlöslichkeit des Extraktionsmittels zunimmt und die Trennung der organischen und der wäßrigen Phase nach der Vorcarbonylierung verbessert wird.

Für das erfindungsgemäße Verfahren geeignete wasserlösliche Polymere sind ferner Polyacrylsäuren oder teilweise oder vollständig neutralisierte Polyacrylsäuren im wesentlichen enthaltend oder bestehend aus Einheiten der allgemeinen Formel 4 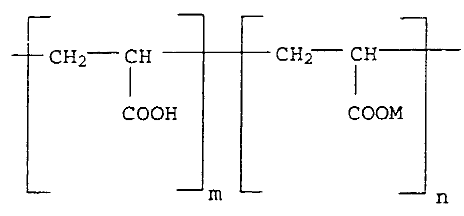 wobei M für Ammonium, für ein Kation eines einwertigen Metalls oder für das Äquivalent eines mehrwertigen Metalls steht, die mittlere Molmasse des wasserlöslichen Polymers von 500 bis 250000 g/mol und das Verhältnis von m zu (n+m) von 0,01 bis 1, bevorzugt von 0,2 bis 0,7 beträgt, sowie wasserlösliche Acrylsäure-Maleinsäure-Copolymeren oder teilweise oder vollständig neutralisierte Acrylsäure-Maleinsäure-Copolymeren, vorzugsweise solche, enthaltend oder bestehend aus Einheiten der allgemeinen Formel 5 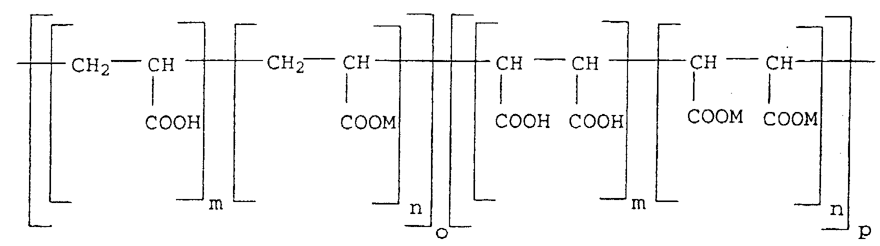 wobei M für Ammonium, für ein Kation eines einwertigen Metalls oder für das Äquivalent eines mehrwertigen Metalls steht, die mittlere Molmasse des wasserlöslichen Polymers von 3000 bis 70000 g/mol und das Verhältnis von m zu (n+m) von 0,01 bis 1, bevorzugt von 0,2 bis 0,7 und das Verhältnis von o zu (o+p) von 0,01 bis 1, bevorzugt von 0,3 bis 0,7 beträgt.

Die ausgezeichnete Eignung der wäßrigen Lösungen der wasserlöslichen Polymeren der genannten Art war überraschend, da sowohl die Beständigkeit gegen die in der Carbonylierung herrschenden hohen Drücke und Temperaturen und der Nichtübergang in die organische Phase nicht zu erwarten war.

Erfindungsgemäß werden im einzelnen die bei der Hydroformylierung mit "nacktem Rhodium" erhaltenen rhodiumhaltigen Hydroformylierungsausträge mit wasserlöslichen Polymeren versetzt, die mit dem Rhodium-Katalysator Komplexe bilden, welche hydrophil sind und infolge ihrer guten Wasserlöslichkeit mit Wasser aus dem organischen Medium des Hydroformylierungsaustrags extrahiert werden können. Nach der extraktiven Abtrennung des im Hydroformylierungsaustrag enthaltenen Rhodium-Katalysators in Form eines wasserlöslichen Komplexes mit dem erfindungsgemäß eingesetzten wasserlöslichen Polymer, kann das Hydroformylierungsprodukt auf an sich übliche Weise aufgearbeitet werden, beispielsweise indem man das Hydroformylierungsprodukt aus dem organischen Extrakt destillativ isoliert oder indem man leichter flüchtige organische Bestandteile des Hydroformylierungsaustrags vom schwerer flüchtigen oder gegebenenfalls sogar undestillierbaren Hydroformylierungsprodukt abdestilliert. Der wäßrige Extrakt des Hydroformylierungsaustrags, welcher den nunmehr vom wasserlöslichen Polymer komplexierten Rhodium-Katalysator enthält, wird, wie in WO 95/25080 beschrieben, einer Vorcarbonylierung zugeleitet, in der der komplexierte Rhodium-Katalysator in Gegenwart einer im wesentlichen wasserunlöslichen, organischen Flüssigkeit und in Gegenwart von Kohlenmonoxid, Synthesegas oder eines Kohlenmonoxid-haltigen Gasgemisches bei einem Druck von im allgemeinen 50 bis 1000 bar, vorzugsweise von 70 bis 500 bar und besonders bevorzugt von 100 bis 400 bar und bei einer Temperatur von 50 bis 180°C, vorzugsweise 70 bis 160°C, insbesondere von 90 bis 140°C, carbonyliert wird. Durch die Carbonylierung wird das Rhodium aus dem hydrophilen Komplex mit dem wasserlöslichen Polymer gelöst, und die sich dabei bildende, lipophile Rhodiumcarbonylverbindung migriert als "nacktes" Rhodium in die wasserunlösliche, organische Flüssigkeit. Dieser Schritt wird als "Vorcarbonylierung" bezeichnet, da die Carbonylierung des Rhodiums nicht im Hydroformylierungsreaktor selbst, sondern in der vorgeschalteten Vorcarbonylierungsstufe erfolgt.

Der Austrag aus der Vorcarbonylierungsstufe kann leicht, z.B. in einem Phasenabschneider, in eine den Hauptteil des Rhodiums in Form einer Rhodiumcarbonylverbindung enthaltende organische Phase und in eine den Hauptteil des wasserlöslichen Polymers enthaltende wäßrige Phase getrennt werden. Die organische Phase wird anschließend der Hydroformylierungsstufe zugeführt, in der das in dieser Katalysatorlösung enthaltene "nackte" Rhodium die Hydroformylierung des zu hydroformylierenden Olefins katalysiert. Die den Hauptteil des wasserlöslichen Polymers enthaltende, wäßrige Phase kann anderweitig verwendet werden, beispielsweise vorteilhaft zur Extraktion des Rhodium-Katalysators aus dem Hydroformylierungsaustrag.

Die Vorcarbonylierung kann mit Hilfe von Kohlenmonoxid, Synthesegas oder eines Kohlenmonoxid-haltigen Gasgemisches durchgeführt werden. Unter "Synthesegas" werden CO/H₂-Gasgemische verstanden, in denen Kohlenmonoxid und Wasserstoff im allgemeinen in einem Molverhältnis von 1:5 bis 5:1, vorzugsweise von 4:6 bis 6:4 vorliegen. Als Kohlenmonoxid-haltige Gasgemische" werden im Sinne dieser Anmeldung andere Kohlenmonoxid-haltige Gasgemische verstanden, die nicht unter den Begriff "Synthesegas" fallen, beispielsweise CO/H₂-Gemische mit von Synthesegas verschiedener Zusammensetzung oder Gemische des Kohlenmonoxids mit anderen, unter den Reaktionsbedingungen inerten Gases, wie Stickstoff, Edelgasen oder niederen Kohlenwasserstoffen, wie Methan, Ethan, Propan oder Butan.

Als im wesentlichen wasserunlösliche organische Flüssigkeit kann erfindungsgemäß eine Vielzahl unter den Reaktionsbedingungen der Vorcarbonylierungsstufe und der Hydroformylierungsstufe inerter Flüssigkeiten verwendet werden, wobei "inert" bedeutet, daß diese Flüssigkeiten nicht den Ablauf der Vorcarbonylierung oder der Hydroformylierung beeinträchtigen.

Als derartige organische Flüssigkeiten können beispielsweise Kohlenwasserstoffe verwendet werden. Vorzugsweise werden aber Aldehyde oder Alkohole oder Gemische aus Aldehyden und Alkoholen verwendet. Beispielsweise kann zu diesem Zweck ein Teil des rohen Austrags aus der Hydroformylierungsstufe verwendet werden, man kann aber auch die in der Hydroformylierungsstufe gebildeten und nachfolgend isolierten Aldehyde oder Alkohole oder deren Gemische verwenden. Allgemein besteht bezüglich der Art der als wasserunlösliche, organische Flüssigkeit in der Vorcarbonylierungsstufe verwendeten Aldehyde praktisch keine Beschränkung. Vorzugsweise werden aber solche Aldehyde oder Alkohole verwendet, wie sie bei der Hydroformylierung des zu hydroformylierenden Olefins entstehen.

Als im wesentlichen wasserunlösliche, organische Flüssigkeit können in der Vorcarbonylierungsstufe auch sogenannte Hochsieder eingesetzt werden. Diese sind hochsiedende Kondensationsprodukte von Aldehyden, die im Zuge der Hydroformylierung als Nebenprodukte entstehen. Es handelt sich hierbei naturgemäß im allgemeinen um Vielkomponentenmischungen. In US-A 4148830 wird die chemische Natur derartiger Hochsiedergemische beispielhaft erläutert. Derartige Hochsiedergemische sind auch im Handel erhältlich; beispielsweise unter der Bezeichnung Texanol® von der Firma Eastman.

Besonders bevorzugt werden Olefine als im wesentlichen wasserunlösliche, organische Flüssigkeit in der Vorcarbonylierungsstufe eingesetzt. Obgleich bezüglich der Art des in der Vorcarbonylierung eingesetzten Olefins prinzipiell keine Beschränkung besteht, werden vorzugsweise solche Olefine eingesetzt, wie sie in der nachfolgenden Hydroformylierungsstufe der Ausgangsolefine verwendet werden.

Es kann sich auch als vorteilhaft erweisen, den gesamten Olefinzulauf für die Hydroformylierungsstufe zunächst durch die Vorcarbonylierungsstufe zu schicken. Wird in der Vorcarbonylierungsstufe Synthesegas als Carbonylierungsgas verwendet, kann das zugeführte Olefin je nach den gewählten Bedingungen in der Vorcarbonylierung bereits in geringen Mengen hydroformyliert werden. Werden Kohlenmonoxid oder Kohlenmonoxid-haltige Gasgemische als Carbonylierungsagens benutzt, so können sich Acyl-Komplexe des carbonylierten Rhodiums mit dem Olefin bilden, wodurch eine zusätzliche Stabilisierung des homogen gelösten Rhodiums erzielt werden kann.

Da beim Einsatz von α-Olefinen in die Vorcarbonylierungsstufe eine Isomerisierung der α-Olefine zu internen Olefinen stattfinden kann, können α-Olefine zu internen Olefinen isomerisiert und diese in der nachfolgenden Hydroformylierungsstufe zu internen, also verzweigten, Aldehyden hydroformyliert werden. Dies ist besonders vorteilhaft, da α-Olefine, verglichen mit internen Olefinen, auf dem Markt in größeren Mengen zur Verfügung stehen als interne Olefine, α-Olefine zudem preiswerter erhältlich sind als interne Olefine und verzweigte Aldehyde und verzweigte Alkohole gesuchte Zwischenprodukte zur Herstellung von verzweigten Carbonsäuren, Alkoholen und Aminen sind, welche wiederum in großem Umfang z.B. als Zusätze für Wasch- und Reinigungsmittel und zur Herstellung von biologisch abbaubaren Tensiden verwendet werden.

Zur Herstellung von verzweigten Alkoholen und/oder Aldehyden aus α-Olefinen wird zweckmäßigerweise der α-Olefinzulauf durch die Vorcarbonylierungsstufe geschickt. Die Vorcarbonylierung und simultan dazu die Isomerisierung des α-Olefins zum internen Olefin wird im allgemeinen bei Temperaturen von 100 bis 180°C, vorzugsweise bei 120 bis 160°C, besonders bevorzugt bei 130 bis 150°C und bei einem Druck von 50 bis 1000 bar, vorzugsweise 70 bis 500 bar und besonders bevorzugt bei 100 bis 400 bar durchgeführt. Die zur vollständigen Isomerisierung des α-Olefins erforderliche Verweilzeit des α-Olefins in der Vorcarbonylierungsstufe ist im allgemeinen von den darin angewandten Reaktionsbedingungen abhängig und wird zweckmäßigerweise durch einen Vorversuch bestimmt.

Selbstverständlich können nach dem vorliegenden Verfahren auch α-Olefine zu n-Aldehyden hydroformyliert werden, beispielsweise indem man das α-Olefin unter Umgehung der Vorcarbonylierungsstufe in den Hydroformylierungsreaktor einleitet oder indem man Reaktionsbedingungen in der Vorcarbonylierungsstufe anwendet, unter denen das zugeführte α-Olefin nicht in nennenswertem Umfang isomerisiert wird.

Die Vorcarbonylierungsstufe kann aus einem oder mehreren, parallel oder hintereinander geschalteten Reaktoren bestehen. Bei einer diskontinuierlichen Betriebsweise können hierzu herkömmliche Rührautoklaven und bei einer kontinuierlichen Betriebsweise Kaskaden-Rührautoklaven oder Rohrreaktoren, die zur Durchmischung des Reaktionsgutes geeignete Vorrichtungen enthalten, verwendet werden.

Der Austrag aus der Vorcarbonylierungsstufe wird in einer geeigneten Vorrichtung, z.B. einem Phasenabschneider, in eine wäßrige und eine organische Phase getrennt. Die Phasenabscheidung kann unter Druck, beispielsweise unter dem Betriebsdruck der Vorcarbonylierungsstufe oder bei Atmosphärendruck, nach vorausgegangener Entspannung des Austrags aus der Vorcarbonylierung erfolgen. Da sowohl die Vorcarbonylierung als auch die Hydroformylierung unter erhöhtem Druck erfolgen, wird die Phasentrennung vorteilhaft ebenfalls unter Druck vorgenommen.

Die so aus dem Austrag der Vorcarbonylierungsstufe abgetrennte organische Phase, die das zur Katalyse der Hydroformylierung benötigte "nackte" Rhodium und je nach Art der in der Vorcarbonylierungsstufe verwendeten wasserunlöslichen, organischen Flüssigkeit das zu hydroformylierende Olefin oder eine andere geeignete organische Flüssigkeit enthält, kann der Hydroformylierungsstufe zugeführt werden. Wurde keine Entspannung und Entgasung des Vorcarbonylierungsaustrags vorgenommen, enthält die organische Phase weiterhin das in der Vorcarbonylierungsstufe verwendete gasförmige Carbonylierungsagens im wesentlichen in gelöster Form.

Die Hydroformylierung wird mit Hilfe des in der Vorcarbonylierungsstufe erzeugten "nackten" Rhodium-Katalysators in Gegenwart von Synthesegas durchgeführt. Erforderlichenfalls wird der Hydroformylierungsstufe noch das zu hydroformylierende Olefin zugeführt, falls dieses der Hydroformylierungsstufe nicht schon mit der organischen Phase aus dem Vorcarbonylierungsaustrag zugeführt wurde.

Die Hydroformylierung wird im allgemeinen bei Temperaturen von 60 bis 180°C, vorzugsweise 80 bis 140°C und besonders bevorzugt bei 90 bis 130°C und bei einem Druck von im allgemeinen 50 bis 1000 bar, vorzugsweise bei 70 bis 500 bar, insbesondere bei 100 bis 400 bar durchgeführt. Die Hydroformylierung erfolgt ansonsten unter Bedingungen, wie sie üblicherweise bei Hydroformylierungen mit "nacktem" Rhodium angewandt werden und wie sie beispielsweise in der eingangs zitierten Literatur, betreffend die Hydroformylierung mit "nacktem" Rhodium, beschrieben sind.

In Abhängigkeit von den in der Hydroformylierungsstufe angewandten Druck- und Temperaturbedingungen und der Synthesegaszusammensetzung kann das Produktverhältnis Alkohol/Aldehyd im Hydroformylierungsaustrag beeinflußt werden. Beispielsweise wird bei der Hydroformylierung von Trimerpropylen bei jeweils gleichen Synthesegaszusammensetzungen - CO/H₂-Molverhältnis 50:50, 40:60 bzw. 60:40 - bei 130°C und einem Druck von 280 bar - ein Aldehyd/ Alkohol-Molverhältnis von jeweils 93:7 erhalten. Bei Erhöhung der Temperatur von 130°C auf 150°C verändert sich das Aldehyd/Alkohol-Molverhältnis im Hydroformylierungsaustrag in Abhängigkeit von der Synthesegaszusammensetzung - CO/H₂-Molverhältnis 50:50, 40:60 bzw. 60:40 - auf 76:24, 67:33 bzw. 82:18.

Die Hydroformylierung kann in An- oder Abwesenheit von organischen Lösungsmitteln durchgeführt werden. Besonders vorteilhaft ist die Verwendung organischer Lösungsmittel, insbesondere bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende Aldehyd-Kondensationsprodukte, die im Zuge der Hydroformylierungsreaktion als Nebenprodukt als Folge der Kondensation der Produktaldehyde entstehen.

Der Austrag aus der Hydroformylierungsstufe wird vor seiner Extraktion mit der wäßrigen Lösung des stickstoffhaltigen Komplexbildners zweckmäßigerweise entspannt. Die Extraktion des Hydroformylierungsaustrags wird im allgemeinen bei Temperaturen von 50 bis 140°C, vorzugsweise von 70 bis 130°C, insbesondere von 90 bis 120°C und bei einem Druck von im allgemeinen 1 bis 20 bar, vorzugsweise 1 bis 10 bar und besonders bevorzugt von 1 bis 5 bar durchgeführt. Die Extraktion kann an der Luft oder unter einer Inertgasatmosphäre durchgeführt werden, beispielsweise einer Stickstoff-, Wasserstoff- oder Argonatmosphäre, es kann aber auch vorteilhaft sein, dem verwendeten Inertgas zusätzlich Kohlenmonoxid oder Synthesegas beizumischen oder die Extraktion in Gegenwart von Kohlenmonoxid durchzuführen.

Zur Extraktion des Rhodium-Katalysators aus dem Hydroformylierungsaustrag kann eine frische wäßrige Lösung des Komplexbildners verwendet werden, vorzugsweise wird hierfür aber die bei der Phasentrennung des Vorcarbonylierungsaustrags erhaltene, den gelösten Komplexbildner enthaltende wäßrige Phase verwendet, die zu diesem Zweck in die Extraktionsstufe zurückgeführt wird.

Bei der Extraktion wird im allgemeinen ein Volumenverhältnis wäßrige/organische Phase von im allgemeinen 0,2 bis 2, vorzugsweise von 0,3 bis 1, eingestellt. Der Gehalt der wäßrigen Phase an wasserlöslichen, polymeren Extraktionsmittel beträgt im allgemeinen 0,1 bis 50%, bevorzugt 1 bis 30% und besonders bevorzugt 3 bis 10%.

Zur Extraktion des Hydroformylierungsaustrags mit der wäßrigen Lösung des wasserlöslichen Polymers sind praktisch alle flüssig-flüssig-Extraktionsapparaturen geeignet, beispielsweise Mixer-Settler-Apparaturen, Blasensäulen oder Gegen- oder Gleichstromextraktionskolonnen, wobei diese noch mit zusätzlichen Einbauten zur besseren Durchmischung von wäßriger und organischer Phase versehen sein können, beispielsweise mit Siebböden, Füllkörpern oder statischen Mischern. Die Extraktion des Rhodium-Katalysators aus dem Hydroformylierungsaustrag kann einstufig durchgeführt werden, vorzugsweise wird eine Mehrstufenextraktion angewandt, beispielsweise eine Zwei- oder Dreistufenextraktion, wobei die wäßrige, den Komplexbildner enthaltende Phase im Gleichstrom oder, besonders bevorzugt, im Gegenstrom bezüglich der organischen Phase geführt werden kann.

Nach beendeter Extraktion kann der von Rhodium-Katalysator befreite Hydroformylierungsaustrag auf an sich herkömmliche Weise, beispielsweise destillativ zur Isolierung der darin enthaltenen Wertprodukte-Alkohole und/oder Aldehyde aufgearbeitet werden.

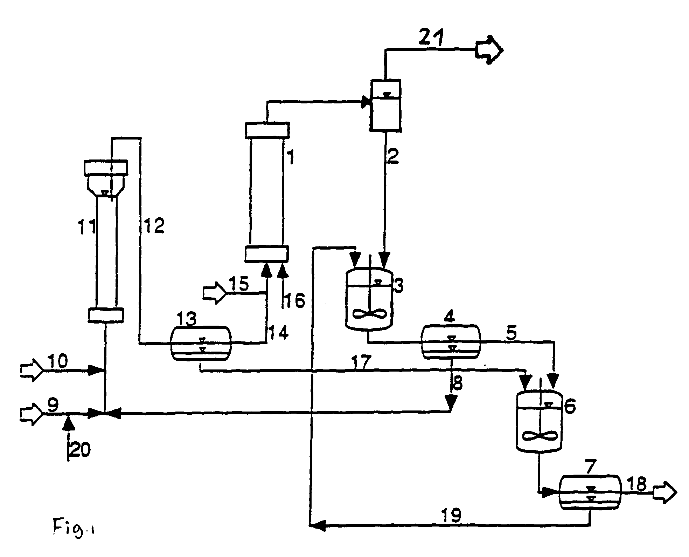

Eine vorteilhafte Ausgestaltung des erfindungsgemäßen Verfahrens wird in Fig. 1 schematisch dargestellt und im folgenden erläutert. An sich selbstverständliche Anlagendetails, die zu der Veranschaulichung des erfindungsgemäßen Verfahrens nicht erforderlich sind, wurden aus Gründen der Übersichtlichkeit nicht in der Fig. 1 aufgenommen. Die in Fig. 1 dargestellte Ausgestaltung des erfindungsgemäßen Verfahrens umfaßt die Verfahrensstufen der Hydroformylierung, eine zweistufige Gegenstromextraktion des Hydroformylierungsaustrags mittels Mixer-Settler-Apparaturen und die Vorcarbonylierungsstufe. Es versteht sich von selbst, daß an Stelle der Mixer-Settler-Apparaturen auch andere der oben erwähnten Extraktionsapparaturen eingesetzt werden können.

In der Ausgestaltung des erfindungsgemäßen Verfahrens gemäß Fig. 1 wird der Hydroformylierungsaustrag aus dem Hydroformylierungsreaktor 1 über Leitung 2 nach Entspannung, Abtrennung der flüssigen Phase von überschüssigem Synthesegas (Abgas) über Leitung 21 und gegebenenfalls nach Zufuhr von Inertgas (nicht eingezeichnet) in die Extraktionsstufe A, umfassend die Mixer-Settler-Apparatur 3/4, eingetragen und dort mit der über Leitung 19 zugeführten wäßrigen Lösung des wasserlöslichen Polymers aus Extraktionsstufe C (Mixer-Settler-Apparatur 6/7) extrahiert. Beim Anfahren der Anlage oder zum Zwecke der Ergänzung der wäßrigen Polymerlösung kann frische Polymerlösung über einen nicht in Fig. 1 eingezeichneten Einlaß, z.B. dem Mixer 3 zugeführt werden. Das im Mixer 3 enthaltene Extraktionsgemisch wird im Settler 4 in eine erste organische und eine erste wäßrige Phase aufgetrennt. Die erste wäßrige Phase wird über Leitung 8 in den Vorcarbonylierungsreaktor 11 geleitet, wohingegen die erste organische Phase über Leitung 5 der Extraktion 6 (Extraktionsstufe C) zugeführt wird. Vor Einleitung in den Vorcarbonylierungsreaktor 11 wird die erste wäßrige Phase noch in geeigneten Mischapparaturen über die Zuläufe 9 und 10 mit einer im wesentlichen wasserunlöslichen, organischen Flüssigkeit, beispielsweise rohem Hydroformylierungsaustrag, Texanol® oder vorzugsweise dem zu hydroformylierenden Olefin und dem Carbonylierungsagens, also Kohlenmonoxid, Synthesegas oder einem geeigneten Kohlenmonoxid-haltigen Gasgemisch, vorzugsweise mit Kohlenmonoxid, Synthesegas, vermischt. Es ist grundsätzlich auch möglich die über die Leitungen 9 und 10 zugeführten Einsatzstoffe direkt in den Vorcarbonylierungsreaktor 11 einzuleiten. Im Vorcarbonylierungsreaktor 11 wird das an das wasserlösliche Polymer gebundene, in der wäßrigen Phase befindliche Rhodium unter den angegebenen Bedingungen carbonyliert und der sich dabei bildende, lipophile "nackte" Rhodium-Katalysator migriert in die organische Phase. Der Austrag aus dem Vorcarbonylierungsreaktor 11 wird über Leitung 12, vorzugsweise ohne vorherige Entspannung, in den Phasenabschneider 13 geleitet und dort in eine zweite organische und eine zweite wäßrige Phase getrennt (Phasenabscheidung B).

Die zweite organische Phase, die außer der wasserunlöslichen, organischen Flüssigkeit noch das zur Katalyse der Hydroformylierung benötigte "nackte" Rhodium und gegebenenfalls überschüssiges Carbonylierungsagens gelöst enthält, wird über Leitung 14 in den Hydroformylierungsreaktor 1 geleitet. Über Leitung 15 wird dem Hydroformylierungsreaktor Synthesegas zugeleitet, welches alternativ auch direkt in Hydroformylierungsreaktor 1 geleitet werden kann. Wurde im Vorcarbonylierungsreaktor als wasserunlösliche, organische Flüssigkeit kein Olefin verwendet, so kann das zu hydroformylierende Olefin entweder direkt über Leitung 16 in den Hydroformylierungsreaktor 1 eingeleitet werden oder vorher über einen nicht in Fig. 1 eingezeichneten Einlaß dem Strom in Leitung 14 zugemischt werden. Im Hydroformylierungsreaktor wird das Olefin unter den angegebenen Bedingungen zu den entsprechenden Alkoholen und/oder Aldehyden hydroformyliert.

Die zweite wäßrige Phase aus der Phasenabscheidung 13 (Phasenabscheidung B), die die an Rhodium abgereicherte Lösung des wasserlöslichen Polymers enthält, wird über Leitung 17 nach vorheriger Entspannung dem Mixer 6 zugeführt. In Extraktionsstufe C, umfassend den Mixer 6 und den Settler 7, wird die erste organische Phase aus Extraktionsstufe A mit der zweiten wäßrigen Phase aus Phasenabscheidung B nochmals extrahiert, um Restmengen von Rhodium aus der ersten organischen Phase zu entfernen. Die im Mixer 6 enthaltene Extraktionsmischung wird im Settler 7 in eine dritte organische und eine dritte wäßrige Phase getrennt. Die nunmehr von Rhodium befreite dritte organische Phase wird über Leitung 18 zur weiteren Aufarbeitung zwecks Isolierung der Wertprodukte - Alkohol und/oder Aldehyd - ausgetragen. Die dritte wäßrige Phase aus Extraktion C wird über Leitung 19 in die Extraktionsstufe A geleitet, womit der Kreislauf geschlossen ist.

Die Erstbefüllung des Reaktors mit Rhodium kann durch Einführung einer Lösung oder Suspension des Rhodiumkatalysators oder eine der zur Herstellung des Rhodium-Katalysators geeigneten, eingangs erwähnten Vorläuferverbindungen, beispielsweise in den Vorcarbonylierungsreaktor 11 oder in den Hydroformylierungsreaktor 1, erfolgen. Gleiches gilt für gegebenenfalls erforderliche Ergänzungen von verbrauchtem Katalysator. Es ist auch möglich das Rhodium über Leitung 20 oder andere, in Fig. nicht eingezeichnete Einlässe, beispielsweise über einen Einlaß an Leitung 8, in die Anlage einzuleiten.

Das erfindungsgemäße Verfahren eignet sich besonders gut zur Hydroformylierung von Olefinen mit mehr als 3, vorzugsweise mehr als 7 Kohlenstoffatomen, insbesondere zur Hydroformylierung von C₇- bis C₂₀-Olefinen, die geradkettig oder verzweigt sein können und die α-olefinische und/oder interne Doppelbindungen enthalten können, z.B. Octen-1, Dodecen-1, Trimer- und Tetramerpropylen, oder Dimer-, Trimer- und Tetramerbutylen. Ebenso können ungesättigte Oligomere anderer Olefine hydroformyliert werden. Desgleichen Cooligomere verschiedener Olefine. Die aus diesen Olefinen gebildeten Aldehyde dienen z.B. als Vorstufen für die Herstellung von Weichmacheralkoholen und Tensiden, die daraus auf an sich herkömmliche Weise durch Hydrierung erzeugt werden können. Die zur Hydroformylierung eingesetzten Olefine können z.B. durch die säurekatalysierte Wasserabspaltung aus den entsprechenden Fettalkoholen oder nach einer Vielzahl anderer technischer Verfahren, wie sie beispielsweise in Weissermel, Arpe: Industrielle Organische Chemie, S. 67-86, Verlag Chemie, Weinheim, 1978, dargestellt sind, erhalten werden. Werden im erfindungsgemäßen Verfahren α-Olefine eingesetzt, so können diese wahlweise durch direkten Eintrag in die Hydroformylierungsstufe zu den entsprechenden n-Aldehyden hydroformyliert oder durch deren Eintrag in die Vorcarbonylierungsstufe, nach ihrer Isomerisierung zu internen Olefinen, zu Isoaldehyden hydroformyliert werden, auf deren Anwendung bereits hingewiesen wurde.

Besonders gut geeignet ist das erfindungsgemäße Verfahren auch zur Hydroformylierung von polymeren Olefinen, beispielsweise niedermolekularen Polyisobuten, niedermolekularem Polybutadien oder niedermolekularem 1,3-Butadien-Isobuten- oder Buten-Copolymeren. Unter "niedermolekulare Polymere" werden insbesondere Polymere mit Molgewichten von 280 bis 5000 Dalton verstanden. Es können aber auch höhermolekulare, ungesättigte Polymere mit Molgewichten von größer 5000 hydroformyliert werden. Einzige Voraussetzung hierfür ist, daß diese im Hydroformlyierungsmedium löslich sind.

Das vorliegende Verfahren eignet sich demgemäß praktisch zur Herstellung aller Aldehyde, die auf dem Wege der Hydroformylierung von Olefinen erhältlich sind. Insbesondere sei darauf hingewiesen, daß beispielsweise auch substituierte Olefine, die im allgemeinen 1 bis 2, vorzugsweise einen Substituenten tragen können, nach dem erfindungsgemäßen Verfahren hydroformyliert werden können. Beispielsweise können nach dem erfindungsgemäßen Verfahren ungesättigte, aliphatische Carbonsäureester, Acetale, Alkohole, Ether, Aldehyde, Ketone, Amine und Amide hydroformyliert werden. Als derartige substituierte Ausgangsolefine sind z.B. Methacrylsäureester, Dicyclopentadien, Vinyl- und Allylether, insbesondere entsprechend substituierte Derivate ungesättigter Fettsäuren von Interesse, beispielsweise die Ester der Öl-, Linol-, Linolen-, Ricinol- oder Erucasäure. Die aus diesen olefinischen Rohstoffen durch Hydroformylierung erhältlichen Aldehyde sind ebenfalls Ausgangsmaterialien zur Herstellung biologisch leicht abbaubarer, waschaktiver Substanzen.

Eine weitere Möglichkeit bietet sich mit einem Verfahren zur Herstellung verzweigter Carbonsäuren, Alkohole oder Amine aus α-Olefinen, wobei die α-Olefine nach dem vorliegenden Verfahren in der Vorcarbonylierungsstufe zu internen Olefinen isomerisiert, anschließend zu Isoaldehyden hydroformyliert und die so erhaltenen Isoaldehyde auf an sich herkömmliche Weise zu verzweigten Carbonsäuren oxidiert, zu verzweigten Alkoholen reduziert oder verzweigten Aminen reduktiv aminiert werden.

Die Oxidation der aus α-Olefinen erhaltenen Isoaldehyde oder Isoaldehyd/n-Aldehyd-Gemische kann auf an sich bekannte Weise, beispielsweise durch die Oxidation der Aldehyde mit Luftsauerstoff oder Sauerstoff gemäß den Verfahren, wie sie z.B. in Ullmann's Encyclopedia of Industrial Chemistry, 5. Aufl., Vol. A5, S. 239, VCH Verlagsgesellschaft, Weinheim, 1986 dargestellt sind, erfolgen.

Die katalytische Hydrierung der nach dem erfindungsgemäßen Verfahren aus α-Olefinen erhältlichen Isoaldehyde oder Isoaldehyd/ n-Aldehydgemische zu verzweigten Alkoholen kann auf an sich bekannte Weise, beispielsweise nach den Verfahren von Ullmann's Encyclopedia of Industrial Chemistry, 5. Aufl., Vol. A1, S. 279, VCH Verlagsgesellschaft, Weinheim, 1985 oder G.H. Ludwig, Hydrocarbon Processing, März 1993, S. 67, bewerkstelligt werden.

Die reduktive Aminierung der nach dem erfindungsgemäßen Verfahren aus α-Olefinen erhältlichen Isoaldehyde oder Isoaldehyd/n-Aldehydgemische kann auf an sich bekannte Weise, beispielsweise nach den Verfahren von Ullmann's Encyclopedia of Industrial Chemistry, 5. Aufl., Vol. A2, S. 1, VCH Verlagsgesellschaft, Weinheim, 1985 erfolgen. Als Ausgangsmaterial zur Herstellung von Aminen können sowohl Ammoniak, primäre C₁- bis C₂₀-Amine oder sekundäre C₂- bis C₂₀-Amine eingesetzt werden.

### Beispiel 1 (phosphonomethyliertes Polyethylenimin, mittlere Molmasse 6500 g/mol, Verhältnis m: (n+m) = 0,9)

### Extraktion:

125 ml eines Austrages aus der Hydroformylierung von Octen-N (einem Isomerengemisch aus Butendimeren) wurden mit 125 ml einer wäßrigen Lösung von phosphonomethylierten Polyethylenimin extrahiert. Von ursprünglich 71 Gew.-ppm Rhodium waren nach der Extraktion noch 4 Gew.-ppm in der organischen Phase.

### Vorcarbonylierung:

75 ml einer wäßrigen, rhodiumhaltigen Extraktionslösung mit phosphonomethyliertem Polyethylenimin wurden mit 75 ml Octen-N in einem Autoklaven bei einem Kohlenmonoxiddruck von 280 bar und einer Temperatur von 130°C 3 Stunden lang gerührt. Dabei wurde das an das wasserlösliche Polymer gebundene Rhodium carbonyliert und in die wäßrige Phase extrahiert. Die organische Phase enthielt 40 Gew.-ppm, die wäßrige Phase 9 Gew.-ppm Rhodium, während das wasserlösliche Polymer in der wäßrigen Phase verblieb.

### Hydroformylierung:

Der Austrag aus dem Vorcarbonylierungsreaktor wurde in eine wäßrige und eine organische Phase getrennt. Die organische Phase wurde anschließend in einem Hydroformylierungsreaktor bei 130°C und einem Synthesegasdruck (CO/H₂-Verhältnis 1:1) von 280 bar hydroformyliert. Der Umsatz bei der Hydroformylierung betrug 93%.

### Beispiel 2 (Polyacrylsäure teilneutralisiert, mittlere Molmasse 1000 g/mol Verhältnis m: (n+m) = 0,5)

### Extraktion:

125 ml eines Austrages aus der Hydroformylierung von Octen-N (einem Isomerengemisch aus Butendimeren) wurden mit 125 ml einer wäßrigen Lösung von teilneutralisierter Polyacrylsäure extrahiert. Von ursprünglich 100 Gew.-ppm Rhodium waren nach der Extraktion noch 6 Gew.-ppm in der organischen Phase.

### Vorcarbonylierung:

75 ml einer wäßrigen; rhodiumhaltigen Extraktionslösung mit teilneutralisierter Polyacrylsäure wurden mit 75 ml Octen-N in einem Autoklaven bei einem Kohlenmonoxiddruck von 280 bar und einer Temperatur von 130°C 3 Stunden lang gerührt. Dabei wurde das an das wasserlösliche Polymer gebundene Rhodium carbonyliert und in die wäßrige Phase 7 Gew.-ppm Rhodium, während das wasserlösliche Polymer in der wäßrigen Phase verblieb.

### Hydroformylierung:

Der Austrag aus dem Vorcarbonylierungsreaktor wurde in eine wäßrige und eine organische Phase getrennt. Die organische Phase wurde anschließend in einem Hydroformylierungsreaktor bei 130°C und einem Synthesegasdruck (CO/H₂-Verhältnis 1:1) von 280 bar hydroformyliert. Der Umsatz bei der Hydroformylierung betrug 97%.

### Beispiel 3

Wie in Fig. 1 und der dazugehörigen Erläuterung beschrieben, wurde ein kontinuierlicher Versuch unter Verwendung von wasserlöslichem phosphonomethyliertem Polyethylenimin, mittlere Molmasse 6500 g/mol, Verhältnis m: (n+M) = 0,9 durchgeführt.

| | |
|---|---|
| Zuläufe/h: 10 | 200 g Isoocten |
| 9 | 20 N-Liter CO |
| 8 | 200 g Katalysatorlösung mit 20 ppm Rhodium |
| 15 | 100 N-Liter Synthesegas |

Temperatur Vorcarbonylierung (11) 100°C
Temperatur Reaktor (1) 120°
Austrag/h: 18: 228 g Austrag mit 0,4 ppm Rhodium

## Patentansprüche

1. Verfahren zur Herstellung von Aldehyden oder Aldehyden und Alkoholen durch Hydroformylierung von Olefinen mit mehr als 3 Kohlenstoffatomen, umfassend eine Hydroformylierungsstufe, in der man mittels eines in einem homogenen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodiumkatalysators mit einer wäßrigen Lösung eines Komplexbildners, Isolierung des Alkohols und/oder Aldehyds aus dem extrahierten Hydroformylierungsaustrag, Vorcarbonylierung des wäßrigen rhodiumhaltigen Extrakts in Gegenwart von Kohlenmonoxid, Synthesegas oder eines Kohlenmonoxid enthaltenden Gasgemisches bei einem Druck von 50 bis 1000 bar und einer Temperatur von 50 bis 180°C, Trennung des Austrags der Vorcarbonylierung in eine den Hauptteil des Rhodiums enthaltende organische Phase und eine den Komplexbildner enthaltende wäßrige Phase und Rückführung der organischen Phase in die Hydroformylierungsstufe, dadurch gekennzeichnet, daß man den Rhodiumkatalysator aus dem Austrag der Hydroformylierungsstufe mit einer wäßrigen Lösung eines sulfonsäuregruppen-freien, wasserlöslichen, das mit dem Rhodiumkatalysator einen wasserlöslichen Polymeren Komplex bildet,extrahiert und aus dem gebildeten wasserlöslichen Komplex das Rhodium in der Vorcarbonylierung als lipophile Rhodiumcarbonylverbindung herauslöst.

2. Verfahren zur Herstellung von Aldehyden oder Aldehyden und Alkoholen durch Hydroformylierung von Olefinen mit mehr als 3 Kohlenstoffatomen gemäß Anspruch 1, umfassend eine Hydroformylierungsstufe, in der man mittels eines in einem homogenen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodiumkatalysators mit einer wäßrigen Lösung eines Komplexbildners, Isolierung des Alkohols und/oder Aldehyds aus dem extrahierten Hydroformylierungsaustrag, Vorcarbonylierung des wäßrigen rhodiumhaltigen Extrakts in Gegenwart von Kohlenmonoxid, Synthesegas oder eines Kohlen monoxid enthaltenden Gasgemisches bei einem Druck von 50 bis 1000 bar und einer Temperatur von 50 bis 180°C, Trennung des Austrags der Vorcarbonylierung in eine dem Hauptteil des Rhodiums enthaltende organische Phase und eine den Komplexbildner enthaltende wäßrige Phase und Rückführung der organischen Phase in die Hydroformylierungsstufe, dadurch gekennzeichnet, daß man den
Rhodiumkatalysator aus dem Austrag der Hydroformylierungsstufe mit einer wäßrigen Lösung eines sulfonsäuregruppenfreien, wasserlöslichen Polymeren extrahiert, ausgewählt aus der Gruppe bestehend aus
(a) Polyacrylsäuren, die teilweise oder vollständig neutralisiert sein können, mit einer mittleren Molmasse von mehr als 500 g/mol,
(b) Maleinsäure-Copolymeren mit einer mittleren Molmasse von mehr als 3000 g/mol,
(c) einfach oder mehrfach phosphonomethylierte Polyvinylaminen mit einer mittleren Molmasse von mehr als 800 g/mol,
(d) einfach oder mehrfach phosphonomethylierte Polyethyleniminen mit einer mittleren Molmasse von mehr als 200 g/mol, und/oder
(e) einfach oder mehrfach phosphonomethylierte Polyacrylamiden mit einer mittleren Molmasse von mehr als 800 g/mol.

3. Verfahren nach Anspruch 1, dadurch gekennzeichnet, daß man als wasserlösliches Extraktionsmittel ein wasserlösliches Salz eines phosphonomethylierten Polyethylenimins verwendet mit Einheiten der Formel 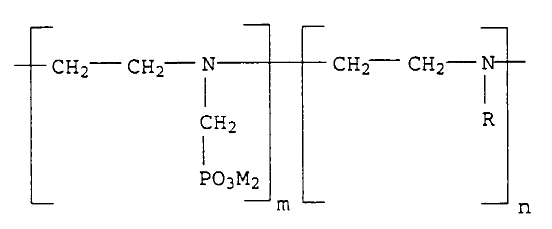 wobei jedes M unabhängig stehen kann für H, Ammonium, ein , einwertiges Metall oder für das Äquivalent eines mehrwertigen Metalls, wobei das mittlere Molekulargewicht des wasserlöslichen Polymers von 200 bis 2000000 g/mol, und das Verhältnis von m zu (n+m) von 0,01 bis 1, bevorzugt von 0,5 bis 1 beträgt, wobei jedes R unabhängig H, Alkyl, Aryl, Hydroxyalkyl oder Carboxyalkyl sein kann.

4. Verfahren nach Anspruch 1, dadurch gekennzeichnet, daß man als wasserlösliches Extraktionsmittel ein wasserlösliches Salz eines phosphonomethylierten Polyvinylamins verwendet mit Einheiten der Formel 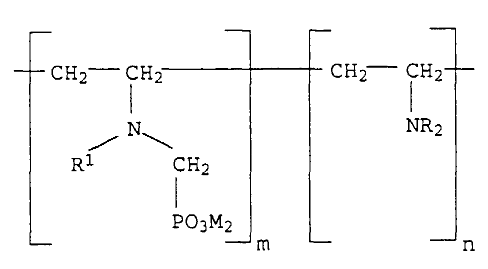 wobei jedes M unabhängig für Wasserstoff, Ammonium, ein Kation eines Metalls oder für das Äquivalent eines mehrwertigen Metalls steht, das mittlere Molekulargewicht des wasserlöslichen Polymers von 800 bis 5000000 g/mol und das Verhältnis von m zu (n+m) von 0,01 bis 1, bevorzugt von 0,5 bis 1 beträgt, R¹ für R oder CH₂PO₃M₂ steht und jedes R unabhängig Wasserstoff, Alkyl, Aryl, Hydroxyalkyl oder Carboxyalkyl sein kann.

5. Verfahren nach Anspruch 1, dadurch gekennzeichnet, daß man als wasserlösliches Extraktionsmittel ein wasserlösliches Salz eines phosphonomethylierten Polyacrylamids verwendet mit Einheiten der Formel 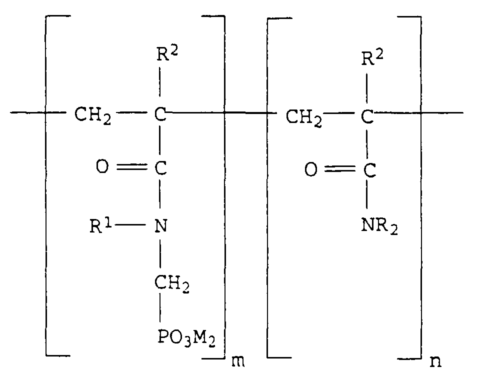 wobei jedes M unabhängig für Wasserstoff, Ammonium, ein Kation eines einwertigen Metalls oder für das Äquivalent eines mehrwertigen Metalls steht, das mittlere Molekulargewicht des wasserlöslichen Polymers von 800 bis 5000000 g/mol und das Verhältnis von m zu (n+m) von 0,01 bis 1, bevorzugt von 0,5 bis 1 beträgt, R¹ für R oder CH₂PO₃M₂, R² für Wasserstoff oder Alkyl steht und jedes R unabhängig Wasserstoff, Alkyl, Aryl, Hydroxyalkyl oder Carboxyalkyl sein kann.

6. Verfahren nach Anspruch 1, dadurch gekennzeichnet, daß man als wasserlösliches Extraktionsmittel Polyacrylsäure oder ein teilweise oder vollständig neutralisiertes Salz einer Polyacrylsäure verwendet mit Einheiten der Formel 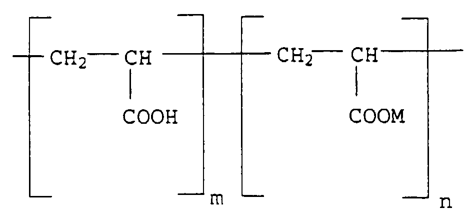 wobei M für Ammonium, für ein Kation eines einwertigen Metalls oder für das Äquivalent eines mehrwertigen Metalls steht, die mittlere Molmasse des wasserlöslichen Polymers von 500 bis 250000 g/mol und das Verhältnis von m zu (n+m) von 0,01 bis 1, bevorzugt von 0,2 bis 0,7 beträgt.

7. Verfahren nach Anspruch 1, dadurch gekennzeichnet, daß man als wasserlösliches Extraktionsmittel ein Acrylsäure-Maleinsäure-Copolymer oder ein teilweise oder vollständig neutralisiertes Salz eines Acrylsäure-Maleinsäure-Copolymers mit Einheiten der Formel 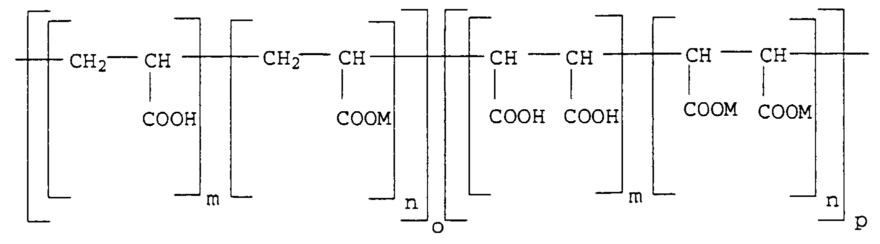 verwendet,
wobei M für Ammonium, ein Kation eines einwertigen Metalls oder für das Äquivalent eines mehrwertigen Metalls steht, die mittlere Molmasse des wasserlöslichen Polymers von 3000 bis 70000 g/mol und das Verhältnis von m zu (n+m) von 0,01 bis 1, bevorzugt von 0,2 bis 0,7 und das Verhältnis von o zu (o+p) von 0,01 bis 1, bevorzugt von 0,3 bis 0,7 beträgt.

8. Verfahren nach Anspruch 1, dadurch gekennzeichnet, daß man in der Vorcarbonylierungsstufe als im wesentlichen wasserunlösliche organische Flüssigkeit den Rohaustrag aus der Hydroformylierungsstufe verwendet.

9. Verfahren nach Anspruch 1, dadurch gekennzeichnet, daß man in der Vorcarbonylierungsstufe als im wesentlichen wasserunlösliche Flüssigkeit das zu hydroformylierende α-Olefin einsetzt und die Vorcarbonylierung bei einem Druck von mehr als 100 bar und bei einer Temperatur von unter 110°C durchführt.

10. Kontinuierliches Verfahren nach Anspruch 1, dadurch gekennzeichnet, daß man den Austrag aus der Hydroformylierungsstufe in einer Extraktionsstufe A mit der wäßrigen Lösung des polymeren Extraktionsmittels aus einer zweiten Extraktionsstufe C extrahiert, dieses Extraktionsgemisch der Extraktionsstufe A in eine erste wäßrige und eine erste organische Phase trennt, die erste wäßrige Phase der Vorcarbonylierungsstufe und die erste organische Phase der Extraktionsstufe C zuführt,
in der Vorcarbonylierungsstufe das in der ersten wäßrigen Phase enthaltene komplexierte Rhodium in Gegenwart einer im wesentlichen wasserunlöslichen, organischen Flüssigkeit mit Kohlenmonoxid, Synthesegas oder einem Kohlenmonoxid-enthaltenden Gasgemisch carbonyliert,
den Austrag aus der Vorcarbonylierungsstufe in einer Phasenabscheidung B in eine zweite organische und eine zweite wäßrige Phase trennt,
die zweite organische Phase dem Hydroformylierungsreaktor und die zweite wäßrige Phase der Extraktionsstufe C zuführt, in der Hydroformylierungsstufe das Olefin in Gegenwart von Synthesegas hydroformyliert,
die zweite wäßrige Phase zur Extraktion von restlichem Rhodium-Katalysator aus der ersten organischen Phase in Extraktionsstufe C verwendet,
das Extraktionsgemisch aus Extraktionsstufe C in eine dritte organische Phase und eine dritte wäßrige Phase trennt, aus der dritten organischen Phase den Aldehyd und/oder Alkohol isoliert,
und die dritte wäßrige Phase zur Extraktion des Rhodium-Katalysators aus dem Hydroformylierungsaustrag in die Extraktionsstufe A zurückführt.

## Claims

1. A process for preparing aldehydes or aldehydes and alcohols by hydroformylation of olefins having more than 3 carbon atoms, comprising a hydroformylation step in which the olefin is hydroformylated at from 50 to 1000 bar and a temperature of from 50 to 180°C by means of a rhodium catalyst dissolved in a homogeneous reaction medium and a catalyst recovery step by extraction of the rhodium catalyst using an aqueous solution of a complexing agent, isolation of the alcohol and/or aldehyde from the extracted hydroformylation product, precarbonylation of the aqueous rhodium-containing extract in the presence of carbon monoxide, synthesis gas or a gas mixture comprising carbon monoxide at a pressure of from 50 to 1000 bar and a temperature of from 50 to 180°C, separation of the product from the precarbonylation into an organic phase containing the major part of the rhodium and an aqueous phase containing the complexing agent and recycling of the organic phase to the hydroformylation step, wherein the rhodium catalyst is extracted from the product from the hydroformylation step using an aqueous solution of a water-soluble polymer which is free of sulfonic acid groups and forms a water-soluble complex with the rhodium catalyst and the rhodium is, in the precarbonylation, leached as a lipophilic rhodium carbonyl compound from the water-soluble complex formed.

2. A process for preparing aldehydes or aldehydes and alcohols by hydroformylation of olefins having more than 3 carbon atoms as claimed in claim 1, comprising a hydroformylation step in which the olefin is hydroformylated at from 50 to 1000 bar and a temperature of from 50 to 180°C by means of a rhodium catalyst dissolved in a homogeneous reaction medium and a catalyst recovery step by extraction of the rhodium catalyst using an aqueous solution of a complexing agent, isolation of the alcohol and/or aldehyde from the extracted hydroformylation product, precarbonylation of the aqueous rhodium-containing extract in the presence of carbon monoxide, synthesis gas or a gas mixture comprising carbon monoxide at a pressure of from 50 to 1000 bar and a temperature of from 50 to 180°C, separation of the product from the precarbonylation into an organic phase containing the major part of the rhodium and an aqueous phase containing the complexing agent and recycling of the organic phase to the hydroformylation step, wherein
the rhodium catalyst is extracted from the product from the hydroformylation step using an aqueous solution of a water-soluble polymer which is free of sulfonic acid groups and is selected from the group consisting of
(a) polyacrylic acids, which may be partially or completely neutralized, having a mean molar mass of more than 500 g/mol,
(b) maleic acid copolymers having a mean molar mass of more than 3000 g/mol,
(c) singly or multiply phosphonomethylated polyvinylamines having a mean molar mass of more than 800 g/mol,
(d) singly or multiply phosphonomethylated polyethylenimines having a mean molar mass of more than 200 g/mol, and/or
(e) singly or multiply phosphonomethylated polyacrylamides having a mean molar mass of more than 800 g/mol.

3. A process as claimed in claim 1, wherein the water-soluble extractant used is a water-soluble salt of a phosphonomethylated polyethylenimine comprising units of the formula 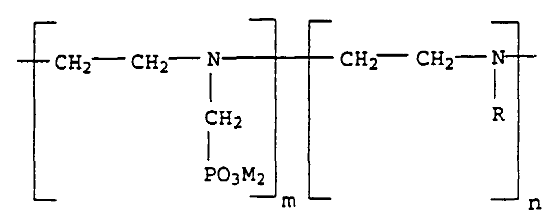 where each M can independently be H, ammonium, a monovalent metal or the equivalent of a polyvalent metal, where the mean molecular weight of the water-soluble polymer can be from 200 to 2,000,000 g/mol and the ratio of m to (n+m) is from 0.01 to 1, preferably from 0.5 to 1, where each R can independently be H, alkyl, aryl, hydroxyalkyl or carboxyalkyl.

4. A process as claimed in claim 1, wherein the water-soluble extractant used is a water-soluble salt of a phosphonomethylated polyvinylamine comprising units of the formula 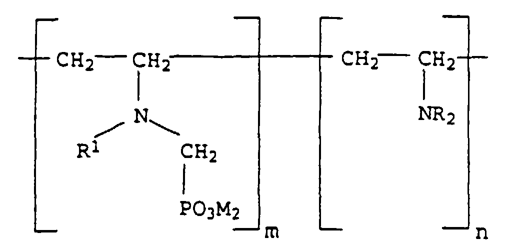 where each M is independently hydrogen, ammonium, a cation of a monovalent metal or the equivalent of a polyvalent metal, the mean molecular weight of the water-soluble polymer is from 800 to 5,000,000 g/mol and the ratio of m to (n+m) is from 0.01 to 1, preferably from 0.5 to 1, R¹ is R or CH₂PO₃M₂ and each R can independently be hydrogen, alkyl, aryl, hydroyalkyl or carboxyalkyl.

5. A process as claimed in claim 1, wherein the water-soluble extractant used is a water-soluble salt of a phosphonomethylated polyacrylamide comprising units of the formula 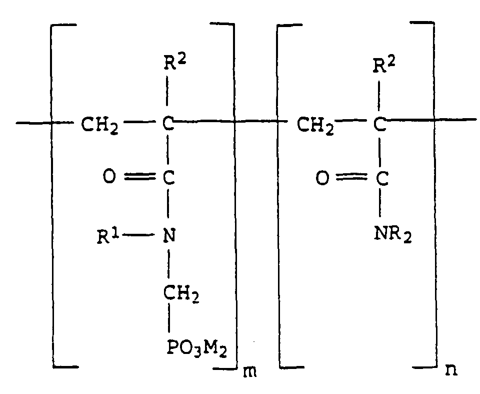 where each M is independently hydrogen, ammonium, a cation of a monovalent metal or the equivalent of a polyvalent metal, the mean molecular weight of the water-soluble polymer is from 800 to 5,000,000 g/mol and the ratio of m to (n+m) is from 0.01 to 1, preferably from 0.5 to 1, R¹ is R or CH₂PO₃M₂, R² is hydrogen or alkyl and each R can independently be hydrogen, alkyl, aryl, hydroxyalkyl or carboxyalkyl.

6. A process as claimed in claim 1, wherein the water-soluble extractant used is polyacrylic acid or a partially or completely neutralized salt of a polyacrylic acid comprising units of the formula 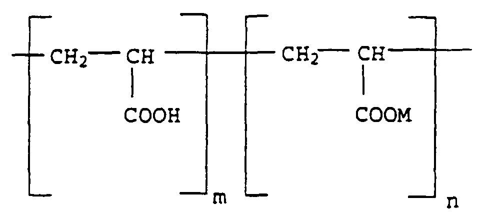 where M is ammonium, a cation of a monovalent metal or the equivalent of a polyvalent metal, the mean molar mass of the water-soluble polymer is from 500 to 250,000 g/mol and the ratio of m to (n+m) is from 0.01 to 1, preferably from 0.2 to 0.7.

7. A process as claimed in claim 1, wherein the water-soluble extractant used is an acrylic acid-maleic acid copolymer or a partially or completely neutralized salt of an acrylic acid-maleic acid copolymer comprising units of the formula 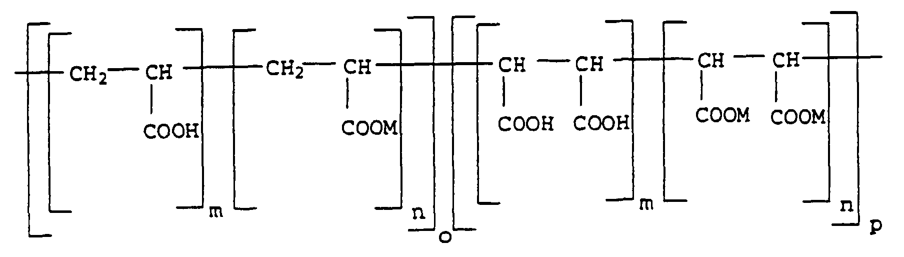 where M is ammonium, a cation of a monovalent metal or the equivalent of a polyvalent metal, the mean molar mass of the water-soluble polymer is from 3000 to 70,000 g/mol and the ratio of m to (n+m) is from 0.01 to 1, preferably from 0.2 to 0.7, and the ratio of o to (o+p) is from 0.01 to 1, preferably from 0.3 to 0.7.

8. A process as claimed in claim 1, wherein the essentially water-insoluble organic liquid used in the precarbonylation step is the crude product from the hydroformylation step.

9. A process as claimed in claim 1, wherein the essentially water-insoluble liquid used in the precarbonylation step is the α-olefin to be hydroformylated and the precarbonylation is carried out at a pressure of more than 100 bar and a temperature of less than 110°C.

10. A continuous process as claimed in claim 1, wherein the product from the hydroformylation step is extracted in an extraction step A with the aqueous solution of the polymeric extractant from a second extraction step C, this extraction mixture from the extraction step A is separated into a first aqueous phase and a first organic phase, the first aqueous phase is fed to the precarbonylation step and the first organic phase is fed to the extraction step C,
in the precarbonylation step, the complexed rhodium present in the first aqueous phase is carbonylated in the presence of an essentially water-insoluble organic liquid using carbon monoxide, synthesis gas or a gas mixture comprising carbon monoxide,
the product from the precarbonylation step is separated into a second organic phase and a second aqueous phase in a phase separation B,
the second organic phase is fed to the hydroformylation reactor and the second aqueous phase is fed to the extraction step C,
in the hydroformylation step, the olefin is hydroformylated in the presence of synthesis gas, the second aqueous phase is used for extracting remaining rhodium catalyst from the first organic phase in the extraction step C,
the extraction mixture from extraction step C is separated into a third organic phase and a third aqueous phase,
the aldehyde and/or alcohol is isolated from the third organic phase,
and the third aqueous phase is recirculated to the extraction step A to extract the rhodium catalyst from the hydroformylation product.

## Revendications

1. Procédé de préparation d'aldéhydes ou d'aldéhydes et d'alcools, par hydroformylation d'oléfines ayant plus de trois atomes de carbone, comprenant une étape d'hydroformylation, dans laquelle on hydroformyle l'oléfine à 50-1000 bar et à une température de 50-180°C, à l'aide d'un catalyseur au rhodium dissous dans un milieu réactionnel homogène, et une étape de récupération du catalyseur par extraction du catalyseur au rhodium avec une solution aqueuse d'un agent complexant, isolement de l'alcool et/ou de l'aldéhyde à partir du produit d'hydroformylation extrait, pré-carbonylation de l'extrait aqueux contenant du rhodium en présence de monoxyde de carbone, de gaz de synthèse ou d'un mélange gazeux contenant du monoxyde de carbone, à une pression de 50-1000 bar et à une température de 50-180°C, séparation du produit de la pré-carbonylation en une phase organique contenant la majeure partie du rhodium et en une phase aqueuse contenant l'agent complexant, et renvoi de la phase organique dans l'étape d'hydroformylation, caractérisé en ce que l'on extrait le catalyseur au rhodium du produit de l'étape d'hydroformylation avec une solution aqueuse d'un polymère hydrosoluble ne comportant pas de groupes (acide sulfonique) et qui forme un complexe hydrosoluble avec le catalyseur au rhodium, et en ce que l'on extrait le rhodium du complexe hydrosoluble formé, par dissolution en tant que composés rhodium-carbonyle lipophiles dans la pré-carbonylation.

2. Procédé de préparation d'aldéhydes ou d'aldéhydes et d'alcools, par hydroformylation d'oléfines ayant plus de trois atomes de carbone selon la revendication 1, comprenant une étape d'hydroformylation, dans laquelle on hydroformyle l'oléfine à 50-1000 bar et à une température de 50-180°C, à l'aide d'un catalyseur au rhodium dissous dans un milieu réactionnel homogène, et une étape de récupération du catalyseur par extraction du catalyseur au rhodium avec une solution aqueuse d'un agent complexant, isolement de l'alcool et/ou de l'aldéhyde à partir du produit d'hydroformylation extrait, pré-carbonylation de l'extrait aqueux contenant du rhodium en présence de monoxyde de carbone, de gaz de synthèse ou d'un mélange gazeux contenant du monoxyde de carbone, à une pression de 50-1000 bar et à une température de 50-180°C, séparation du produit de la pré-carbonylation en une phase organique contenant la majeure partie du rhodium et en une phase aqueuse contenant l'agent complexant, et renvoi de la phase organique dans l'étape d'hydroformylation, caractérisé en ce que l'on extrait le catalyseur au rhodium du produit de l'étape d'hydroformylation avec une solution aqueuse d'un polymère hydrosoluble ne comportant pas de groupes (acide sulfonique) choisi dans le groupe formé par
(a) les poly(acide acrylique)s pouvant être totalement ou partiellement neutralisés, ayant une masse molaire moyenne de plus de 500 g/mole,
(b) les copolymères d'acide maléique, ayant une masse molaire moyenne de plus de 3000 g/mole,
(c) les poly(vinylamine)s une ou plusieurs fois phosphonométhylées, ayant une masse molaire moyenne de plus de 800 g/mole,
(d) les poly(éthylène-imine)s une ou plusieurs fois phosphonométhylées, ayant une masse molaire moyenne de plus de 200 g/mole, et/ou
(e) les poly(acrylamide)s une ou plusieurs fois phosphonométhylés, ayant une masse molaire moyenne de plus de 800 g/mole.

3. Procédé selon la revendication 1, caractérisé en ce que l'on utilise en tant qu'agent d'extraction hydrosoluble un sel hydrosoluble d'une poly(éthylène-imine) phosphonométhylée ayant des motifs de formule 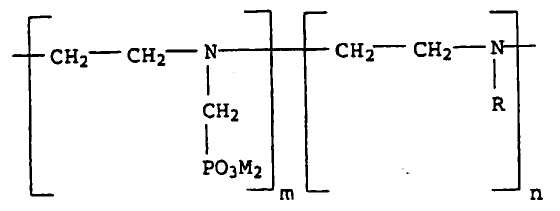 où chaque M représentent indépendamment les uns des autres H, un groupe ammonium, un cation d'un métal monovalent ou l'équivalent d'un métal polyvalent, où la masse moléculaire moyenne du polymère hydrosoluble est de 200-2000000 g/mol, et le rapport de m à (n+m) est de 0,01-1, de préférence 0,5-1, où chaque R peut représenter indépendamment des autres, H un groupe alkyle, aryle, hydroxyalkyle ou carboxyalkyle.

4. Procédé selon la revendication 1, caractérisé en ce que l'on utilise en tant qu'agent d'extraction hydrosoluble un sel hydrosoluble d'une poly(vinylamine) phosphonométhylée ayant des motifs de formule 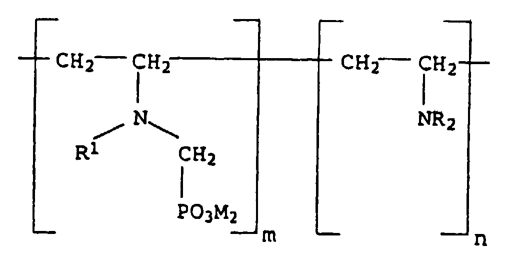 où chaque M représentent indépendamment les uns des autres H, un groupe ammonium, un cation d'un métal monovalent ou l'équivalent d'un métal polyvalent, où la masse moléculaire moyenne du polymère hydrosoluble est de 800-5000000 g/mol, et le rapport de m à (n+m) est de 0,01-1, de préférence 0,5-1, R¹ est mis pour R ou CH₂PO₃M₂ et chaque R peut représenter indépendamment des autres, H, un groupe alkyle, aryle, hydroxyalkyle ou carboxyalkyle.

5. Procédé selon la revendication 1, caractérisé en ce que l'on utilise en tant qu'agent d'extraction hydrosoluble un sel hydrosoluble d'un poly(acrylamide) phosphonométhylé ayant des motifs de formule 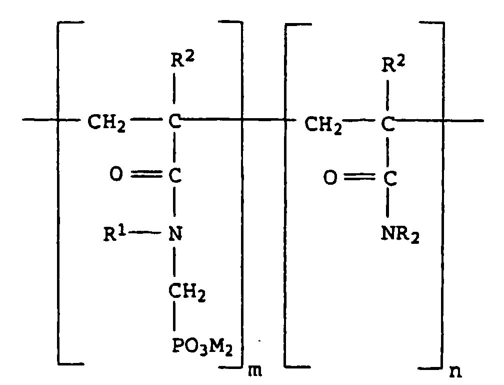 où chaque M représentent indépendamment les uns des autres H, un groupe ammonium, un cation d'un métal monovalent ou l'équivalent d'un métal polyvalent, où la masse moléculaire moyenne du polymère hydrosoluble est de 800-5000000 g/mol, et le rapport de m à (n+m) est de 0,01-1, de préférence 0,5-1, R¹ est mis pour R ou CH₂PO₃M₂, R² est mis pour un atome d'hydrogène ou un groupe alkyle et chaque R peut représenter indépendamment des autres, H, un groupe alkyle, aryle, hydroxyalkyle ou carboxyalkyle.

6. Procédé selon la revendication 1, caractérisé en ce que l'on utilise en tant qu'agent d'extraction hydrosoluble, du poly(acide acrylique) ou un sel totalement ou partiellement neutralisé d'un poly(acide acrylique) ayant des motifs de formule 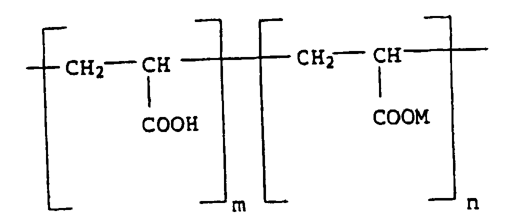 où M représente un groupe ammonium, un cation d'un métal monovalent ou l'équivalent d'un métal polyvalent, où la masse moléculaire moyenne du polymère hydrosoluble est de 500-250000 g/mol, et le rapport de m à (n+m) est de 0,01-1, de préférence 0,2-0,7.

7. Procédé selon la revendication 1, caractérisé en ce que l'on utilise en tant qu'agent d'extraction hydrosoluble un copolymère acide acrylique/acide maléique ou un sel totalement ou partiellement neutralisé d'un copolymère acide acrylique/acide maléique ayant des motifs de formule 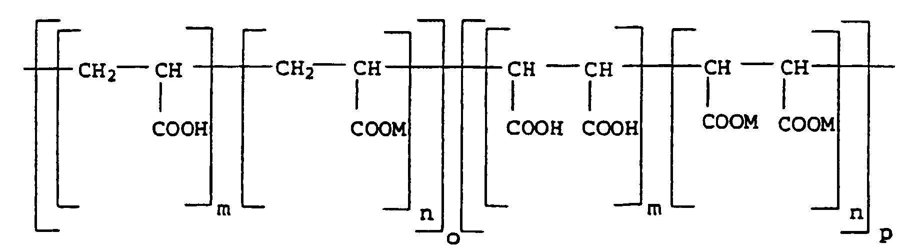 où M représente un groupe ammonium, un cation d'un métal monovalent ou l'équivalent d'un métal polyvalent, où la masse moléculaire moyenne du polymère hydrosoluble est de 3000-70000 g/mol, et le rapport de m à (n+m) est de 0,01-1, de préférence 0,2-0,7, et le rapport de o à (o+p) est de 0,01-1, de préférence de 0,3-0,7.

8. Procédé selon la revendication 1, caractérisé en ce que l'on utilise dans l'étape de pré-carbonylation, en tant que liquide organique quasiment insoluble dans l'eau, le produit brut de l'étape d'hydroformylation.

9. Procédé selon la revendication 1, caractérisé en ce que l'on utilise dans l'étape de pré-carbonylation, en tant que liquide quasiment insoluble dans l'eau, l'a-oléfine à hydroformyler et l'on effectue la pré-carbonylation sous une pression de plus de 100 bar et à une température de moins de 110°C.

10. Procédé en continu selon la revendication 1, caractérisé en ce que l'on extrait le produit de l'étape d'hydroformylation dans une étape d'extraction A avec la solution aqueuse de l'agent d'extraction polymère d'une deuxième étape d'extraction C,
on divise ce mélange d'extraction de l'étape d'extraction A en une première phase aqueuse et en une première phase organique,
on amène la première phase aqueuse à l'étape de pré-carbonylation et la première phase organique à l'étape d'extraction C,
on carbonyle le rhodium complexé contenu dans la première phase aqueuse, dans l'étape de pré-carbonylation, en présence d'un liquide organique quasiment insoluble dans l'eau, avec du monoxyde de carbone, du gaz de synthèse ou un mélange gazeux contenant du monoxyde de carbone,
on divise le produit de l'étape de pré-carbonylation dans une séparation de phase B en une deuxième phase organique et en une deuxième phase aqueuse,
on amène la deuxième phase organique au réacteur d'hydroformylation et la deuxième phase aqueuse à l'étape d'extraction C,
on hydroformyle l'oléfine en présence de gaz de synthèse dans l'étape d'hydroformylation,
on utilise la deuxième phase aqueuse dans l'étape d'extraction C pour l'extraction du catalyseur au rhodium restant à partir de la première phase organique,
on divise le mélange d'extraction de l'étape d'extraction C en une troisième phase organique et en une troisième phase aqueuse,
on isole l'aldéhyde et/ou l'alcool de la troisième phase organique,
et on renvoie la troisième phase aqueuse dans l'étape d'extraction A pour l'extraction du catalyseur au rhodium à partir du produit d'hydroformylation.
